# EUROPEAN PATENT SPECIFICATION

(11) **EP 2 910 213 B1**
(45) Date of publication and mention of the grant of the patent: **20.03.2019**
(21) Application number: 13840133.6
(22) Date of filing: 28.03.2013
(51) Int. Cl.: A61B 18/14, A61N 1/05

(54) **MULTI-POLE SYNCHRONOUS RADIOFREQUENCY ABLATION CATHETER FOR PULMONARY ARTERY**
MEHRPOLIGER KATHETER ZUR ABLATION MITTELS SYNCHRONER FUNKFREQUENZEN FÜR LUNGENARTERIEN
CATHÉTER D'ABLATION PAR RADIOFRÉQUENCE SYNCHRONE MULTIPOLAIRE POUR ARTÈRE PULMONAIRE

(30) Priority: 13.11.2012 CN 201210453470; 27.03.2013 CN 201310103141
(43) Date of publication of application: 26.08.2015
(73) Proprietor: Pulnovo Medical (Wuxi) Co., Ltd., Xishan Economic Development Zone Wuxi Jiangsu 214191 (CN)
(72) Inventor: CHEN, Shaoliang, Nanjing, Jiangsu 210006 (CN)
(74) Representative: ABG Intellectual Property Law, S.L.
(86) International application number: PCT/CN2013/073338
(87) International publication number: WO 2014/075415

(56) References cited:
- WO-A2-01/37723
- WO-A2-01/37925
- WO-A2-2004/039273
- WO-A2-2011/139589
- CN-A- 102 198 015
- CN-A- 102 641 153
- CN-A- 102 686 179
- CN-A- 102 908 191
- CN-U- 202 982 207
- US-A1- 2006 241 366
- US-A1- 2008 281 312
- US-A1- 2010 222 859
- US-A1- 2011 160 720
- US-A1- 2012 116 382

## Description

### FIELD OF THE INVENTION

The invention relates to a medical device that treats pulmonary hypertension in the pulmonary artery by an exemplary de-sympathetic method, in particular to a multi-pole synchronous pulmonary artery radiofrequency ablation catheter.

### BACKGROUND OF THE INVENTION

Pulmonary hypertension is an intractable diseases in the cardiovascular, respiratory, connective tissue, immune and rheumatic systems. Clinical treatment means of pulmonary hypertension are limited and therapy efficacy thereof is poor. Incidence of primary pulmonary hypertension is low but those secondary to pulmonary interstitial fibrosis, connective tissue disease, portal hypertension, chronic pulmonary artery embolism and left heart system disorder are common, with five-year mortality rate up to 30%. Therefore, prevention and treatment for pulmonary hypertension is of great significance.

In recent years, new targeted drugs keep emerging based on the researches on the pathogenesis of pulmonary hypertension. However, these drugs have serious limitations including many side effects, inappropriate dosage form, expensive cost and unreliable efficacy, thereby they cannot be widely applied in clinical treatment. Experimental data demonstrates that pulmonary hypertension is associated with hyper sympathetic activity in pulmonary artery and hyper active of the baroreceptor. Blocking the sympathetic nerves in the pulmonary artery or permanently damaging the baroreceptor structure and function thereof can decrease the pulmonary artery pressure, which will be the breakthrough technology in the treatment of pulmonary hypertension. However, this new technique lacks relevant specialized surgical instruments, especially a specialized pulmonary artery radiofrequency ablation catheter.

The documents WO 01/37925 A2, WO 01/37723 A2, US 2012/116382 A1, US 2011/160720 A1, US 2010/222859 A1, US 2008/281312 A1 and WO 2011/139589 A2 disclose devices for a similar purpose.

### SUMMARY OF THE INVENTION

### Technical Problems

The present invention provides a multi-pole synchronous pulmonary artery radiofrequency ablation catheter for treatment of pulmonary hypertension in the pulmonary artery by an exemplary de-sympathetic method that is not part of the invention. The catheter only heats the adherent tissue rather than the blood. It also uses cold saline perfusion to protect the vascular intima, and possesses advantages of simple operation, short operation time and controllable precise ablation.

### Technical Solutions

A multi-pole synchronous pulmonary artery radiofrequency ablation catheter comprises a control handle, a catheter body and an annular ring. The control handle is provided with an adjustment apparatus; the catheter body is hollow, in which a cavity is arranged; a lead wire, a temperature sensing wire and a pull wire are arranged in the cavity; one end of the catheter body is flexible, and the flexible end is connected to the annular ring; the other end of the catheter body is connected to the control handle. One end of the pull wire is connected to the flexible end, and the other end of the pull wire is connected to the adjustment apparatus, the tension of the pull wire is adjusted through the adjustment apparatus to achieve radian control of the flexible end; a shape memory wire is arranged in the annular ring, and one end of the shape memory wire extends to the end of the annular ring and the other end of the shape memory wire passes through the root of the annular ring and is fixed on the flexible end of the catheter body; the annular ring is provided with an electrode group with each electrode connected to the lead wire and the temperature sensing wire; the lead wire and the temperature sensing wire goes through the catheter body and are electrically connected to the control handle.

An infusion tube is arranged in the cavity of the catheter body and a through hole is arranged on the electrode. The infusion tube is connected to the electrode through the annular ring. The transfused fluid flows out from the through hole.

The electrode on the annular ring is made of a material selected from the group consisted of platinum-iridium alloy, gold, stainless steel and nickel alloy, with the number in the range of 3-30 electrodes, a diameter in the range of 1.3-2.0mm, a length in the range of 1.2-4mm and an edge space between adjacent electrodes in the range of 0.5-10mm.

The flexible end of the catheter body is arranged with a counterbore, an inner diameter of the counterbore fits an outer diameter of the root of the annular ring, the root of the annular ring is inserted and fixed into the counterbore.

The flexible end of the catheter body is provided with a groove in which a connector is arranged, one end of the connector is connected to the pull wire and the other end of the connector is connected to the shape memory wire.

The material of the shape memory wire in the annular ring is a shape memory alloy selected from the group consisted of nickel titanium alloy, stainless steel or titanium, with a diameter of 0.25-0.5mm. The diameter of the annular ring is 12-40mm. Preferably, 10 electrodes are arranged on the annular ring. The width of naked section of the electrode is 0.75mm, and the space therebetween is 5mm.

The flexible end is made of medical polymer materials with a length in the range of 30-80mm.

The connection is achieved by a UV-curing adhesive.

The joint between the flexible end and the annular ring is sealed.

The pull wire is made of stainless steel or nickel-titanium alloy. The outside of pull wire is provided with a spring coil, and the outside of the spring coil is provided with a spring sleeve made of polyimide material.

The invention is limited by the scope of the appended independent claim. Further preferred embodiments are disclosed in the dependent claims.

### Beneficial Effects

Owing to the abovementioned technical solutions applied by the present invention, the catheter only radiofrequency heats the adherent tissue rather than the blood, and possesses advantages of simple operation, short operation time and controllable precise ablation. The catheter body is preferably made of a polymer material, which is a poor heat conductor, so that it can avoid transmitting the heat when heating the electrodes to the flowing blood contacting the catheter body, thereby effectively avoid heating the blood. Furthermore, the radian of the flexible end can be controlled by operating the adjustment apparatus, which allows the operator to control the handle with one hand, so as to adjust the radian of the flexible end easily, thereby the electrode on the annular ring can fully adhere to the pulmonary artery and achieve ablation of pulmonary artery intima. During the radiofrequency current generating, the electrodes can produce high local temperature and cause severe damage on the vascular intima. Therefore, the present invention uses cold saline perfusion to cool down the local temperature. When electrode generates current, the saline is automatically and uniformly diffused through the through holes, decreasing the local temperature to be below 60 °C, thereby protect the vascular intima.

### BRIEF DESCRIPTION OF THE DRAWINGS

Figure 1 is a schematic structural diagram of the invention;
Figure 2 is a partially enlarged view of Part B of the present invention;
Figure 3 is schematic sectional view of A-A 'of the present invention;
Figure 4 is a schematic structural view of the outer surface of electrode of the present invention.

In these figures, the following elements have been used:
- 1: catheter body,
- 2: control handle,
- 3: flexible end,
- 4: annular ring,
- 5: electrode,
- 6: lead wire,
- 7: temperature sensing wire,
- 8: pull wire,
- 9: hollow cavity,
- 10: lead wire cavity,
- 11: connector,
- 12: shape memory wire,
- 13: spring coil,
- 14: spring sleeve,
- 15: through hole.

### DETAILED DESCRIPTION OF THE PREFERRED EMBODIMENTS

The following examples further illustrate the present invention, but should not be considered as to limit the present invention. Modifications or replacement of the conditions of the present invention may still fall under the scope of the present invention.

If not otherwise specified, the technical means used in the embodiments are conventional means well known by a person skilled in the art.

### Example 1:

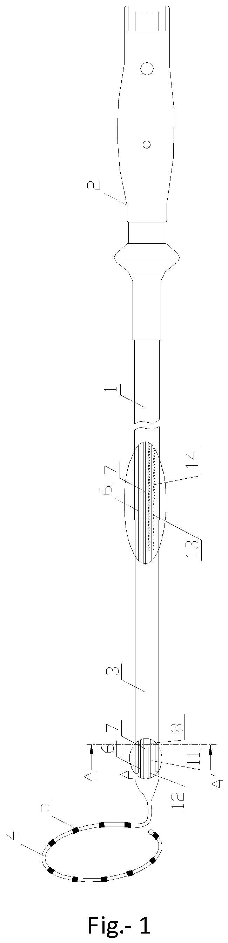

Through the example below and with reference to Figs 1-3, the technical solutions of the present invention are further described in details..

The present invention provides a multi-pole synchronous pulmonary artery radiofrequency ablation catheter for de-sympathetic in the pulmonary artery, wherein a catheter body has a distal end and a proximal end, the distal end is provided with a flexible end and the proximal end is provided with a control handle. A pull wire extends in the catheter body. Preferably, the catheter body is made of a polymer material, which is a poor heat conductor, so that it can avoid transmitting the heat when heating the electrodes to the flowing blood contacting the catheter body, thereby can better prevent the electrode from heating the blood flow. The flexible end includes a proximal end and a distal end, an annular ring is arranged on the distal end, the flexible end is soft relative to the rest of the catheter body; the annular ring is provided with a plurality of electrodes, each electrode being able to extract neural electrical signals, sensing temperature and conduct ablation; each of the electrodes is connected to lead wires and temperature sensing wires, and extends through the catheter body to the control handle, thus is electrically connected to the control handle; a temperature sensing wire is embedded under each electrode for precise monitoring of the temperature during ablation. A shape memory wire is arranged in the annular ring, and a distal end of the shape memory wire extends to the distal end of the annular ring and the proximal end of the shape memory wire is fixed to the distal end of the flexible end. The shape memory wire in the annular ring is preferably made of shape memory alloy which preferably are nickel-titanium alloy, stainless steel or titanium, with a diameter in the range of 0.25-0.5mm. The diameter of the annular ring is in the range of 12-40mm. The length of the flexible end is in the range of 30-80mm, which is made of medical polymer materials such as fluorine, polyesters, polyurethane, polyamide and polyimide. A counterbore is arranged on the distal end of the flexible end, the proximal end of the annular ring is fixed in the counterbore, wherein the proximal end of the annular ring is a ground thin end. A pull wire is embedded in the catheter body, and one end of the pull wire is fixed to the control handle. Radian of the flexible end can be controlled by operating the control handle. For example, one end of the pull wire can be fixed to a control button on the handle and the radian of the flexible end can be controlled by operating the button. This allows the operator to control the handle with one hand and adjust the flexible end easily, so that the electrodes on the annular ring can fully adhere to the pulmonary artery and achieve ablation of pulmonary artery intima.

Furthermore, a counterbore can be made on the distal end of the flexible end 3, and its depth can be set according to actual needs, preferably with a depth in the range of 2-8mm. The proximal end of the annular ring 4 is a ground thin end, and an outer diameter of the ground thin end fits an inner diameter of the counterbore. The ground-thin end is inserted into the flexible end 3 and is fixed to the distal end of the flexible end 3 by bonding, welding or other suitable means, preferably by UV-curing adhesive. The excess glue may be used to seal the distal end of the flexible end 3 and the proximal end of the annular ring 4.

Figure 1 shows a schematic structural diagram of multi-pole synchronous pulmonary artery radiofrequency ablation catheter. The annular ring 4 is arranged at the distal end of the flexible end 3. The annular ring 4 is in annular structure, the radian of the annular ring 4 can be made of shape memory wire. The annular ring 4 is provided with a plurality of electrodes 5. Each electrode is able to extract neural electrical signals, sense the temperature and conduct ablation. The number of electrodes can vary from the range of 3 to 30, preferably 5 to 20. The electrodes are made of platinum-iridium alloy, gold, stainless steel or nickel alloy. The electrode diameter is generally 1.3-2.0mm, and the length of the electrode is generally in the ragne of 1.2-4mm, more suitably 2-3.5mm. Edge space between the adjacent electrodes suitably is in the range of 0.5-10mm, more suitably 1-5mm.

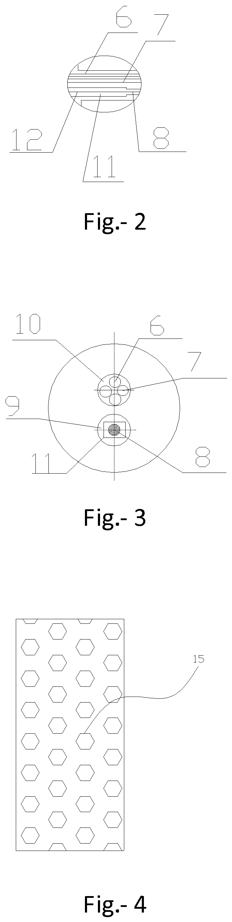

The pull wire 8 is preferably made of stainless steel or nickel-titanium. As shown in Figure 2 and Figure 3. The distal end of the pull wire 8 extends through a hollow cavity 9 to the proximal end of the annular ring 4, and is fixed to the distal end of the flexible end 3. The fixing method can be any known method in the prior art. Preferably, a groove is arranged on the distal end of the flexible end 3, and a connector 11 is arranged in the groove. One end of the connector 11 is connected to the pull wire 8 and the other end of the connector 11 is connected to the shape memory wire 12. The connector 3 is fixed to the distal end of the flexible end 3 by injecting glue such as UV-curing adhesive into the groove. A segment of pull wire 8 extends in the flexible end 3 and a segment of pull wire 8 extends in the catheter body 1. The pull wire is preferably jacketed with a spring coil 13, and the spring coil is jacketed with a spring sleeve 14. The spring sleeve 14 may be made of any suitable material, preferably a polyimide material.

The proximal end of the pull wire 8 is fixed on the control handle 2, which is provided with an adjustment apparatus, and the adjustment apparatus is able to adjust the radian of the flexible end.

Lead wire 6, as shown in Figures 2 and 3, extends through the lead wire cavity 10 to the lead wire cavity of the annular ring 4. The distal end of the lead wire 6 is connected to electrode 5. The distal end of the lead wire 6 is fixed to electrode 5 by welding. The distal end of the temperature sensing wire 7 is embedded under the electrode 5 and the distal end of the temperature sensing wire 7 is fixed on electrode 5 by bonding, welding or other suitable means. The temperature sensing wire 7 extends into the catheter body 1 in the lead wire cavity 10 of the flexible end 3 and then extends out from the control handle 2and is connected to an temperature control device.

When using the catheter, the pull wire 8 is operated through the control handle 2 in order to deflect the flexible end 3, thereby send the annular ring 4 to the orifice of pulmonary artery. Then the electrodes 5 fully adhere to the pulmonary artery. At this time, the electrodes 5 may perform ablation on pulmonary artery intima.

The multi-electrode design according to the present invention can improve the efficacy and safety of ablation, achieve signal analysis and preferable simultaneous ablation by a plurality of electrodes. This can improve target accuracy, achieve timely judgment of ablation effect and save operation time.

### Example 2

A multi-pole synchronous pulmonary artery radiofrequency ablation catheter comprises a control handle 2, a catheter body 1, and an annular ring 4. The control handle 2 is provided with an adjustment apparatus; the catheter body 1 is hollow, and a cavity is arranged in the catheter body 1. A lead wire 6, a temperature sensing wire 7 and a pull wire 8 are arranged in cavity. One end of catheter body is flexible, and the flexible end 3 is connected to the annular ring 4. The other end of the catheter body is connected to the control handle 2. One end of the pull wire 8 is connected to the flexible end 3, and the other end of the pull wire 8 is connected to the adjustment apparatus of the control handle, the adjustment apparatus adjusts the tension of the pull wire to control the radian of the flexible end. This allows the operator to control the handle with one hand and adjust the radian of the flexible end easily. Thereby the electrodes of the annular ring may fully adhere to the pulmonary artery, so as to realize ablation of pulmonary artery intima. A shape memory wire 12 is arranged in the annular ring. One end of the shape memory wire 12 extends to the end of the annular ring 4, and the other end of the shape memory wire 12 goes through the root of the annular ring and is fixed on the flexible end 3 of the catheter body. The annular ring is provided with an electrode group. Each electrode 5 is connected to the lead wire and temperature sensing wire 7 and is able to extract the nerve electrical signals, sense the temperature and conduct ablation. The lead wire 6 and temperature sensing wire 7 go through the catheter body 1 and are electrically connected to the control handle 2. The control handle 2 can be connected to an external temperature control device.

The annular ring electrode is made of a material selected from the group consisited of platinum-iridium alloy, gold, stainless steel and nickel alloy material, with the number in the range of 3-30, a diameter in the range of 1.3-2.0mm, a length in the range of 1.2-4mm and an edge space between adjacent electrodes in the range of 0.5-10mm.

The flexible end of the catheter body has a counterbore. An outer diameter of the root of the annular ring fits an inner diameter of the counterbore. The root of the annular ring is inserted into the counterbore and fixed.

The flexible end 3 of the catheter body is provided with a groove. A connector 11 is arranged in the groove. One end of the connector is connected to the pull wire 8 and the other end of the connector is connected to the shape memory wire 12.

The shape memory wire is made of shape memory alloy such as nickel titanium alloy, stainless steel or titanium, with a diameter in the range of 0.25-0.5mm. The diameter of the annular ring is in the range of 12-40mm. Preferably, 10 electrodes are arranged on the annular ring, and the width of naked section of electrodes is 0.75mm, and the space therebetween is 5mm.

The flexible end of the catheter body is made of medical polymer materials such as fluorine, polyesters, polyurethane, polyamide and polyimide, with a length in the range of 30mm to 80mm.

The connection is via UV-curing adhesive.

The joint between the flexible end of the catheter body and the annular ring is sealed.

The pull wire is made of stainless steel or nickel-titanium alloy. The pull wire is jacketed with a spring coil 13, and the spring coil is jacketed with a spring sleeve 14 made of polyimide material.

### Example 3

Example 3 is similar to Example 1 and Example 2, and the differences are: an infusion tube is arranged in the catheter body; a group of evenly distributed through holes are arranged on the electrode, with a bore diameter of 1µm; one end of the infusion tube is connected to the electrode through the annular ring, and the fluid diffuses out from the through holes on the electrode. The other end of the infusion tube is connected to the transfusion system (a constant-flux pump can be selected). When electrode generates current, the liquid automatically diffuses from the through holes. The transfused liquid can be saline. The cold saline (4 °C) perfusion can help decrease local temperature. When the electrode generates current, the saline can automatically diffuses from the through holes, allow the local temperature to be below 60 °C and thereby protect the vascular intima.

## Claims

1. A multi-pole synchronous pulmonary artery radiofrequency ablation catheter, comprising
a control handle (2),
a catheter body (1), and
an annular ring (4);
the control handle (2) being connected to an external temperature control device and provided with an adjustment apparatus; the catheter body (1) being hollow, with a cavity being arranged in the catheter body; and a lead wire (6), a temperature sensing wire (7) and a pull wire (8) being arranged in the cavity;
wherein:
one end of the catheter body is flexible, and this flexible end (3) is connected to the annular ring (4), and the other end of the catheter body is connected to the control handle (2);
one end of the pull wire (8) is connected to the flexible end (3), and the other end of the pull wire (8) is connected to the adjustment apparatus on the control handle (2), the adjustment apparatus adjusts the tension of the pull wire to control the radian of the flexible end (3);
a shape memory wire (12) is arranged in the annular ring (4), one end of this shape memory wire extending to the end of the annular ring (4) and the other end of the shape memory wire passing through the root of the annular ring (4), being fixed on the flexible end (3) of the catheter body;
the flexible end (3) comprises a groove, with a connector (11) being arranged in the groove, one end of the connector being connected to the pull wire (8) and the other end of the connector being connected to the shape memory wire (12);
the annular ring (4) is provided with an electrode group, the electrode group having a plurality of electrodes (5) provided along the annular ring (4), and each electrode (5) is connected to the lead wire and the temperature sensing wire (7);
the lead wire (6) and temperature sensing wire (7) go through the catheter body (1) and are electrically connected to the control handle (2);
an infusion tube is arranged in the cavity of the catheter body and a through hole (15) is arranged on each electrode (5); the infusion tube going through the annular ring and being connected to each electrode (5); the transfused fluid flowing out from the through holes (15) on the electrodes (5) of the electrode group;
each electrode (5) is able to extract neural electrical signals, sense the temperature and conduct ablation energy; and
when the electrodes (5) receive current, the fluid automatically diffuses from the through holes (15).

2. The multi-pole synchronous pulmonary artery radiofrequency ablation catheter according to claim 1, wherein the electrode on the annular ring is made of a material selected from the group consisting of platinum-iridium alloy, gold, stainless steel and nickel alloy, with a number in the range of 3-30 electrodes, an electrode diameter in the range of 1.3-2.0mm, a length in the range of 1.2-4mm and an edge space between adjacent electrodes in the range of 0.5-10mm.

3. The multi-pole synchronous pulmonary artery radiofrequency ablation catheter according to claim 1, wherein the flexible end (3) of the catheter body (1) is provided with a counterbore, an inner diameter of the counterbore fitting an outer diameter of root of the annular ring, the root of the annular ring being inserted and fixed in the counterbore.

4. The multi-pole synchronous pulmonary artery radiofrequency ablation catheter according to claim 1, wherein the material of the shape memory wire (12) is a shape memory alloy selected from the group consisting of nickel titanium alloy, stainless steel and titanium, with a diameter in the range of 0.25-0.5mm; the diameter of the annular ring (4) being in the range of 12-40mm.

5. The multi-pole synchronous pulmonary artery radiofrequency ablation catheter according to claim 1, wherein the flexible end of the catheter body (1) is made of medical polymer materials with a length in the range of 30-80mm.

6. The multi-pole synchronous pulmonary artery radiofrequency ablation catheter according to any one of claims 1 to 5, wherein the connections are achieved by a UV-curing adhesive.

7. The multi-pole synchronous pulmonary artery radiofrequency ablation catheter according to claim 1, wherein the joint between the flexible end (3) of the catheter body (1) and the annular ring (4) is sealed.

8. The multi-pole synchronous pulmonary artery radiofrequency ablation catheter according to claim 1, wherein the pull wire (8) is made of stainless steel or nickel-titanium alloy; the pull wire being provided with a spring coil (13), and the spring coil being provided with a spring sleeve (14) made of a polyimide material.

9. The multi-pole synchronous pulmonary artery radiofrequency ablation catheter according to claim 1, wherein a group of evenly distributed through holes (15) is arranged on each electrode (5), and wherein the transfused fluid flows out from the group of evenly distributed through holes (15) on each of the electrodes (5) of the electrode group.

10. The multi-pole synchronous pulmonary artery radiofrequency ablation catheter according to claim 1, wherein the fluid automatically diffuses from the through holes (15) to maintain a local temperature of below 60 °C.

## Patentansprüche

1. Mehrpoliger synchroner Radiofrequenzablationskatheter der Pulmonalarterie, umfassend
einen Bediengriff (2),
einen Kathetergehäuse (1), und
einen ringförmigen Ring (4);
der Bediengriff (2) ist mit einem externen Temperaturkontrollgerät verbunden und mit einer Einstellvorrichtung versehen;
das Kathetergehäuse (1) ist hohl, wobei ein Hohlraum im Kathetergehäuse angeordnet ist; und ein Führungsdraht (6), ein temperaturabtastendem Draht (7) und ein Zugdraht (8) sind in dem Hohlraum angeordnet;
wobei:
ein Ende des Kathetergehäuses flexibel ist, und dieses flexible Ende (3) mit dem ringförmigen Ring (4) verbunden ist, und das andere Ende des Kathetergehäuses mit dem Bediengriff (2) verbunden ist;
ein Ende des Zugdrahts (8) mit dem flexiblen Ende (3) verbunden ist, und das andere Ende des Zugdrahts (8) mit der Einstellvorrichtung auf dem Bediengriff (2) verbunden ist, die Einstellvorrichtung die Spannung des Zugdrahts einstellt, um den Radiant des flexiblen Endes (3) zu steuern;
ein Formgedächtnisdraht (12) im ringförmigen Ring (4) angeordnet ist, ein Ende dieses Formgedächtnisdrahts zum Ende des ringförmigen Rings (4) verlängert ist, und das andere Ende des Formgedächtnisdrahtes durch den Fußpunkt des ringförmigen Rings verläuft und am flexiblen Ende (3) des Kathetergehäuses befestigt ist;
das flexible Ende (3) eine Nut umfasst, mit einem Verbindungsstück (11) in der Nut angeordnet, ein Ende des Verbindungsstücks mit dem Zugdraht (8) und
das andere Ende des Verbindungsstücks mit dem Formgedächtnisdraht (12) verbunden ist;
der ringförmige Ring (4) mit einer Elektrodengruppe ausgestattet ist, die Elektrodengruppe eine Vielzahl von Elektroden (5) aufweist, die entlang des ringförmigen Rings (4) bereitgestellt sind, und jede Elektrode (5) mit dem Führungsdraht und dem temperaturabtastendem Draht (7) verbunden ist;
der Führungsdraht (6) und der temperaturabtastende Draht (7) das Kathetergehäuse (1) durchlaufen und elektrisch mit dem Bediengriff (2) verbunden sind;
ein Infusionsschlauch in dem Hohlraum des Kathetergehäuses angeordnet ist und eine Durchgangsöffnung (15) auf jeder Elektrode (5) angeordnet ist; der Infusionsschlauch den ringförmigen Ring durchläuft und mit jeder Elektrode (5) verbunden ist; die eingeleitete Flüssigkeit aus den Durchgangsöffnungen (15) auf die Elektroden (5) der Elektrodengruppe herausfließt;
jede Elektrode (5) in der Lage ist, neurale elektrische Signale zu extrahieren, die Temperatur abzutasten und Ablationsenergie zu leiten; und
wenn die Elektroden (5) einen Strom empfangen, die Flüssigkeit automatisch aus den Durchgangsöffnungen (15) diffundiert.

2. Mehrpoliger synchroner Radiofrequenzablationskatheter der Pulmonalarterie gemäß Anspruch 1, wobei die Elektrode auf dem ringförmigen Ring aus einem Material hergestellt ist ausgewählt aus der Gruppe bestehend aus einer Platin-Iridium-Legierung, Gold, einer Legierung aus rostfreiem Stahl und Nickel, mit einer Anzahl im Bereich von 3-30 Elektroden, einem Elektrodendurchmesser von 1,3-2,0 mm, einer Länge im Bereich von 1,2-4 mm und einer Randlücke zwischen benachbarten Elektroden im Bereich von 0,5-10mm.

3. Mehrpoliger synchroner Radiofrequenzablationskatheter der Pulmonalarterie gemäß Anspruch 1, wobei das flexible Ende (3) des Kathetergehäuses (1) mit einer Senkbohrung versehen ist, der innere Durchmesser der Senkbohrung an den äußeren Durchmesser des Fußpunkts des ringförmigen Rings angepasst ist, der Fußpunkt des ringförmigen Rings in die Senkbohrung eingeführt und fixiert ist.

4. Mehrpoliger synchroner Radiofrequenzablationskatheter der Pulmonalarterie gemäß Anspruch 1, wobei das Material des Formgedächtnisdrahts (12) eine Formgedächtnislegierung ausgewählt aus der Gruppe bestehend aus einer Nickel-Titan-Legierung, rostfreiem Stahl und Titan, mit einem Durchmesser im Bereich von 0,25-0,5mm ist; der Durchmesser des ringförmigen Rings (4) im Bereich von 12-40mm ist.

5. Mehrpoliger synchroner Radiofrequenzablationskatheter der Pulmonalarterie gemäß Anspruch 1, wobei das flexible Ende des Kathetergehäuses (1) aus medizinischen Polymermaterialien mit einer Länge im Bereich von 30-80mm gemacht ist.

6. Mehrpoliger synchroner Radiofrequenzablationskatheter der Pulmonalarterie gemäß einem der Ansprüche 1 bis 5, wobei die Verbindungen durch einen UVhärtenden Klebstoff hergestellt sind.

7. Mehrpoliger synchroner Radiofrequenzablationskatheter der Pulmonalarterie gemäß Anspruch 1, wobei die Verbindung zwischen dem flexiblen Ende (3) des Kathetergehäuses (1) und dem ringförmigen Ring (4) versiegelt ist.

8. Mehrpoliger synchroner Radiofrequenzablationskatheter der Pulmonalarterie gemäß Anspruch 1, wobei der Zugdraht (8) aus rostfreiem Stahl oder einer Nickel-Titan-Legierung gemacht ist; der Zugdraht mit einer Spiralfeder (13) ausgestattet ist, und die Spiralfeder mit einer Federhülle (14), die aus einem Polyimid-Material gemacht ist, ausgestattet ist.

9. Mehrpoliger synchroner Radiofrequenzablationskatheter der Pulmonalarterie gemäß Anspruch 1, wobei eine Gruppe gleichmäßig verteilter Durchgangsöffnungen (15) auf jeder der Elektroden (5) angeordnet ist, und wobei die durchgeleitete Flüssigkeit aus der Gruppe der gleichmäßig verteilten Durchgangsöffnungen (15) auf jeder der Elektroden (5) der Elektrodengruppe austritt.

10. Mehrpoliger synchroner Radiofrequenzablationskatheter der Pulmonalarterie gemäß Anspruch 1, wobei die Flüssigkeit automatisch aus den Durchgangsöffnungen (15) diffundiert, um eine lokale Temperatur von weniger als 60°C aufrecht zu erhalten.

## Revendications

1. Un cathéter d'ablation par radiofréquence de l'artère pulmonaire synchrone multipolaire, comprenant
une poignée de commande (2),
un corps de cathéter (1), et
un anneau annulaire (4) ;
la poignée de commande (2) étant reliée à un dispositif externe de régulation de température et étant munie d'un appareil de réglage ;
le corps du cathéter (1) étant creux, une cavité étant agencée dans le corps du cathéter ; et un fil conducteur (6), un fil de détection de température (7) et un fil de traction (8) étant agencés dans la cavité ;
dans lequel :
une extrémité du corps du cathéter est flexible et cette extrémité flexible (3) est reliée à l'anneau annulaire (4), et l'autre extrémité du corps du cathéter est reliée à la poignée de commande (2) ;
une extrémité du fil de traction (8) est reliée à l'extrémité flexible (3), et l'autre extrémité du fil de traction (8) est reliée à l'appareil de réglage sur la poignée de commande (2), l'appareil de réglage réglant la tension du fil de traction pour commander le radian de l'extrémité flexible (3) ;
un fil à mémoire de forme (12) est disposé dans l'anneau annulaire (4), une extrémité de ce fil à mémoire de forme s'étendant jusqu'à l'extrémité de l'anneau annulaire (4) et l'autre extrémité du fil à mémoire de forme traversant la racine de l'anneau annulaire (4), étant fixée sur l'extrémité flexible (3) du corps du cathéter ;
l'extrémité flexible (3) comprend une rainure, un connecteur (11) étant disposé dans la rainure, une extrémité du connecteur étant reliée au fil de traction (8) et l'autre extrémité du connecteur étant reliée au fil à mémoire de forme (12) ;
l'anneau annulaire (4) est muni d'un groupe d'électrodes, le groupe d'électrodes ayant une pluralité d'électrodes (5) prévues le long de l'anneau annulaire (4), et chaque électrode (5) est connectée au fil conducteur et au fil de détection de température (7) ; le fil conducteur (6) et le fil de détection de température (7) passent à travers le corps du cathéter (1) et sont connectés électriquement à la poignée de commande (2) ;
un tube de perfusion est disposé dans la cavité du corps du cathéter et un trou traversant (15) est aménagé sur chaque électrode (5) ; le tube de perfusion traversant l'anneau annulaire et étant relié à chaque électrode (5) ; le fluide transfusé s'écoulant des trous traversants (15) sur les électrodes (5) du groupe d'électrodes ;
chaque électrode (5) est apte à extraire des signaux électriques neuronaux, de détecter la température et de conduire l'énergie d'ablation ; et
lorsque les électrodes (5) reçoivent du courant, le fluide se diffuse automatiquement depuis les trous traversants (15).

2. Le cathéter d'ablation par radiofréquence de l'artère pulmonaire synchrone multipolaire selon la revendication 1, dans lequel l'électrode sur l'anneau annulaire est constituée d'un matériau choisi dans le groupe constitué par un alliage platine-iridium, de l'or, de l'acier inoxydable et de l'alliage de nickel, avec un nombre compris entre 3 et 30 électrodes, un diamètre d'électrode compris entre 1,3 et 2,0 mm, une longueur comprise entre 1,2 et 4 mm et un intervalle de bordure entre des électrodes adjacentes situé dans l'intervalle allant de 0,5 à 10 mm.

3. Le cathéter d'ablation par radiofréquence de l'artère pulmonaire synchrone multipolaire selon la revendication 1, dans lequel l'extrémité flexible (3) du corps de cathéter (1) est munie d'un contre-perçage, un diamètre intérieur du contre-perçage correspondant à un diamètre extérieur de la racine de l'anneau annulaire, la racine de l'anneau annulaire étant introduite et fixée dans le contreperçage.

4. Le cathéter d'ablation par radiofréquence de l'artère pulmonaire synchrone multipolaire selon la revendication 1, dans lequel le matériau du fil à mémoire de forme (12) est un alliage à mémoire de forme choisi dans le groupe constitué par un alliage de nickel et de titane, un acier inoxydable et du titane, avec un diamètre compris dans l'intervalle allant de 0,25 à 0,5 mm ; le diamètre de l'anneau annulaire (4) étant dans l'intervalle allant de 12 à 40 mm.

5. Le cathéter d'ablation par radiofréquence de l'artère pulmonaire synchrone multipolaire selon la revendication 1, dans lequel l'extrémité flexible du corps de cathéter (1) est constituée de matériaux polymères médicaux ayant une longueur située dans l'intervalle allant de 30 à 80 mm.

6. Le cathéter d'ablation par radiofréquence de l'artère pulmonaire synchrone multipolaire selon l'une quelconque des revendications 1 à 5, dans lequel les connexions sont réalisées par un adhésif durcissant aux UV.

7. Le cathéter d'ablation par radiofréquence de l'artère pulmonaire synchrone multipolaire selon la revendication 1, dans lequel la jonction entre l'extrémité flexible (3) du corps de cathéter (1) et l'anneau annulaire (4) est étanche.

8. Le cathéter d'ablation par radiofréquence de l'artère pulmonaire synchrone multipolaire selon la revendication 1, dans lequel le fil de traction (8) est réalisé en acier inoxydable ou en alliage nickel-titane ; le fil de traction étant muni d'une bobine à ressort (13), et la bobine à ressort étant munie d'un manchon à ressort (14) fait d'un matériau polyimide.

9. Le cathéter d'ablation par radiofréquence de l'artère pulmonaire synchrone multipolaire selon la revendication 1, dans lequel un groupe de trous traversants (15) répartis uniformément est agencé sur chaque électrode (5), et dans lequel le fluide transfusé s'écoule hors du groupe de trous traversants (15) répartis uniformément sur chacune des électrodes (5) du groupe d'électrodes.

10. Le cathéter d'ablation par radiofréquence de l'artère pulmonaire synchrone multipolaire selon la revendication 1, dans lequel le fluide diffuse automatiquement depuis les trous traversants (15) pour maintenir une température locale inférieure à 60°C.
